Europäisches Patentamt

European Patent Office

Office européen des brevets

(11) Veröffentlichungsnummer: **0 183 901 B1**

## EUROPÄISCHE PATENTSCHRIFT

(45) Veröffentlichungstag der Patentschrift: **08.07.92**

(21) Anmeldenummer: **85105814.9**

(22) Anmeldetag: **11.05.85**

(51) Int. Cl.⁵: **G01N 33/531**, G01N 33/94, G01N 33/78, G01N 33/546

(54) Bifunktionelle Haptene, Verfahren zu ihrer Herstellung und ihre Verwendung.

(30) Priorität: **27.11.84 US 675374**

(43) Veröffentlichungstag der Anmeldung:
**11.06.86 Patentblatt 86/24**

(45) Bekanntmachung des Hinweises auf die
Patenterteilung:
**08.07.92 Patentblatt 92/28**

(84) Benannte Vertragsstaaten:
**AT BE CH DE FR GB IT LI LU NL SE**

(56) Entgegenhaltungen:
**EP-A- 0 080 614**
**EP-A- 0 086 095**
**EP-A- 0 149 405**
**DE-A- 2 743 445**

**J. Mol. Biol. (1967), 27, 615-617.**

(73) Patentinhaber: **BEHRINGWERKE Aktiengesellschaft
Postfach 1140
W-3550 Marburg 1(DE)**

Patentinhaber: **HOECHST CELANESE CORPO-
RATION**

**Route 202-206 North
Somerville, N.J. 08876(US)**

(72) Erfinder: **Grenner, Gerd, Dr.
Höhenweg 72
W-3550 Marburg(DE)**
Erfinder: **Kapmeyer, Wolfgang, Dr.
Reinhardswaldstrasse 5
W-3550 Marburg 7(DE)**
Erfinder: **Primes, Kathleen Jelich
Valdina Way 4823
San Diego California 92124(US)**
Erfinder: **Sigler, Gerald Francis
Seri Street 4126
San Diego California 92117(US)**

(74) Vertreter: **Becker, Heinrich Karl Engelbert, Dr.
et al
HOECHST AKTIENGESELLSCHAFT Central
Patent Department P.O. Box 80 03 20
W-6230 Frankturt am Main 80(DE)**

## Beschreibung

Die Erfindung betrifft zweiwertige Haptenderivate der Formel I

A-X-A      I

worin A eine Haptenkomponente und X ein bifunktionelles Brückenglied der Formel II

$(B)_m$-Y-$(CH_2)_n$-Z-$(CH_2)_n$-Y-$(B)_m$      II

bedeuten, worin die Indices m unabhängig voneinander 0 oder 1 sind, B $(CH_2)_{n'}$ mit n' als ganze Zahl von 1 bis 4 oder $CO(CH_2)_{n''}$ mit n'' als ganze Zahl von 2 bis 4 bedeutet,
die Reste Y unabhängig voneinander -CONH-, -NHCO-, -OOC-, -COO-, -O-, -S- oder -NR- sind, wobei R für Wasserstoff oder einen aliphatischen Rest mit bis zu 6 C-Atomen steht,
n eine ganze Zahl von 1 bis 10 und
Z -O-$(CH_2)_4$-O-, Piperaz -1,4-diyl oder -NH-$(CH_2)_4$-NH-ist, ein Verfahren zu ihrer Herstellung sowie ein Verfahren zur Haptenbestimmung mittels eines solchen Derivats.

Ein bifunktionelles Hapten läßt sich als Untergruppe von Hapten/Trägerkonjugaten betrachten. Der Träger ist über zwei funktionelle Gruppen kovalent an die Haptene gebunden. Die beiden Funktionen können verschieden sein, sind aber im allgemeinen gleich. Das Trägermolekül kann ein beliebiges Molekulargewicht und eine beliebige Molekülgestalt aufweisen.

Aus EP-A 0 086 095 ist ein bivalenter Spacer bekannt, wobei die funktionellen Gruppen durch ein geradkettigen Kohlenwasserstoff der Formel -$(CH_2)_n$- mit n = 2 - 10 verbunden sind.

Die hier beschriebenen Träger haben primär die Aufgabe, die beiden Haptenkomponenten durch ein chemisches Brückglied zu trennen. Dank ihres zweiwertigen Charakters können sie zwei Antikörpermoleküle binden, die auf das Hapten ansprechende Bindungsstellen (Epitope) besitzen, wodurch sie sich von freiem Hapten unterscheiden.

Das Konzept zweiwertiger Haptene zur Überbrückung benachbarter Antikörpermoleküle wurde für eine Reihe 2,4-dinitrophenylierter Polymethylendiamine untersucht (J. Molecular Biol., 27, 615, 1967). Es wurde gefunden, daß ein Brückenglied mit einer Mindestlänge von acht Kohlenstoffatomen eine Überbrückung der Antikörper ermöglichte. Es zeigte sich jedoch, daS die Wasserlöslichkeit der beschriebenen Brückenglieder sehr schlecht war. Ein in Wasser besser lösliches zweiwertiges Haptensystem ist erforderlich und erwünscht.

Gegenstand dieser Erfindung ist ein bifunktionelles Haptenderivat.

Insbesondere ist Gegenstand dieser Erfindung ein Derivat, das aus zwei über ein bifunktionelles Brückenglied verbundenen Haptenkomponenten besteht, wodurch das Derivat der Formel I

A-X-A      I

entspricht, worin A eine Haptenkomponente und X ein bifunktionelles Brückenglied der Formel II

$(B)_m$-Y-$(CH_2)_n$-Z-$(CH_2)_n$-Y-$(B)_m$      II

bedeuten, worin die Indices m unabhängig voneinander 0 oder 1 sind, B $(CH_2)_{n'}$ mit n' als ganze Zahl von 1 bis 4 oder $CO(CH_2)_{n''}$ mit n'' als ganze Zahl von 2 bis 4 bedeutet,
die Reste Y unabhängig voneinander -CONH-, -NHCO-, -OOC-, -COO-, -O-, -S- oder -NR- sind, wobei R für Wasserstoff oder einen aliphatischen Rest mit bis zu 6 C-Atomen steht,
n eine ganze Zahl von 1 bis 10 und
Z -O-$(CH_2)_4$-O-, Piperaz -1,4-diyl oder -NH-$(CH_2)_4$-NH-ist.

X ist vor allem eine synthetische chemische Verbindung.

Vorzugsweise ist R Wasserstoff.

Die beschriebenen Verbindungen stellen eine neue Klasse zweiwertiger Haptene dar, in denen der Träger (hier einfach als bifunktionelles Brückenglied bezeichnet) ein homobifunktionelles, vorwiegend lineares, organisches Molekül ist, in dem ein oder mehrere Kohlenstoffatome durch Heteroatome ersetzt sind. Beispiele für bevorzugte Heteroatome sind Sauerstoff und Stickstoff. Der Einbau dieser Heteroatome verleiht den Haptenkonjugaten eine deutlich erhöhte Löslichkeit in Wasser gegenüber Analogen, bei denen diese fehlen. Bei hydrophoben Haptenen ermöglicht diese löslich machende Eigenschaft des Brückenglieds eine Zubereitung der zweiwertigen Haptene in wässrigen Puffern, die zur Bindung von Antikörpern geeignet ist.

Haptene lassen sich mit dem bifunktionellen Brückenglied über verschiedene kovalente Bindungen kuppeln, doch besteht eine besonders bevorzugte Methode in der Herstellung einer Amidbindung. Dazu muß das Hapten entweder eine Carboxylfunktion enthalten oder leicht in diese umwandelbar sein. Ein Beispiel für ein geeignetes, eine Carboxylfunktion enthaltendes Hapten ist L-Thyroxin. Zu Haptenen, in die sich eine Carboxylgruppe einführen läßt, gehören beispielsweise Theophyllin, Phenytoin und Phenobarbital.

In den Fällen, wo das zu kuppelnde Hapten keine Carboxylfunktion enthält, läßt sich diese nach verschiedenen, dem Fachmann bekannten Methoden einführen. Zum Beispiel kann man Haptene, die eine nukleophile Funktion wie sie Amine, Alkohole oder Thiole enthalten, mit einer Halogenalkylcarbonsäure alkylieren oder mit Bernsteinsäureanhydrid zu einem Halbsuccinat umsetzen.

Wenn Aminfunktionen oder sonstige reaktive Gruppen im Hapten vorhanden sind, wie im Fall der Aminosäuren, so müssen diese durch eine Schutzgruppe maskiert werden, bevor eine Kondensation mit einem bifunktionellen Diaminbrückenglied unternommen wird. Die Schutzgruppen können unter allen im Stand der Technik wohlbekannten ausgewählt werden, solange ihre spätere Entfernung nach der Konjugation das zweiwertige Hapten unversehrt läßt. Zu den geeigneten Schutzgruppen zählen t-Butoxycarbonyl, Benzyloxycarbonyl und Trifluoracetyl. Geeignete Maskiermethoden sind im Stand der Technik wohlbekannt.

Das bifunktionelle Brückenglied hat eine solche Länge, daß sterische Wechselwirkungen in der Reaktion mit Antikörperteilchen so gering wie möglich werden. Zudem wählt man das bifunktionelle Brückenglied so aus, daß es Eigenschaften aufweist, die dem zweiwertigen Konjugat Wasserlöslichkeit verleihen, zum Beispiel durch Einbau hydrophiler Atome wie Stickstoff oder Sauerstoff. Deshalb wählt man als Komponente Z des bifunktionellen Brückenglieds die Gruppe -O-(CH$_2$)$_4$-O-, Piperaz-1,4-diyl (1,4-disubstituiertes Piperazin) oder -NH-(CH$_2$)$_4$-NH-, um dem Konjugat Wasserlöslichkeit zu verleihen. Mit diesem Merkmal besitzen die beschriebenen Verbindungen einen Vorteil vor denen dem Stand der Technik bekannten (J.Mol.Biol., 27 615, 1967). Es ist zwar für die Erfindung nicht erforderlich, daß das bifunktionelle Brückenglied symmetrisch ist, doch sind solche bequemer zu synthetisieren. Geeignete bifunktionelle Brückenglieder sind leicht nach dem Stand der Technik wohlbekannten Methoden synthetisierbar und großenteils im Handel erhältlich.

Die Kupplung eines Haptencarboxylats mit einem bifunktionellen Diaminbrückenglied erfolgt durch Voraktivierung der Carbonsäuregruppe nach verschiedenen, zur Bildung von Amidbindungen im Stand der Technik wohlbekannten Methoden. Dazu gehört die Verwendung von Kondensationsmitteln wie Carbodiimiden oder Carbonyldiimidazol sowie die Überführung der Carboxygruppe in ein Anhydrid, Säurechlorid oder einen aktiven Ester. Eine ungewöhnliche aktivierte Carboxylfunktion des Theophyllins ist das cyclische Lactam des 8-Buttersäurederivats (Beispiel 5).

Die Kupplung des aktivierten Haptencarboxylats an das bifunktionelle Diaminbrückenglied erfolgt bei einem Mindestmolverhältnis von 2 Haptenen pro bifunktionelles Brückenglied und vorzugsweise mit einem mäßigen Überschuß der Haptenkomponente, um eine vollständige Reaktion zu erzielen. Zum Kuppeln lassen sich verschiedene Lösungsmittel einsetzen. Besonders bevorzugt sind Lösungsmittel des dipolaren aprotischen Typs wie Dimethylformamid oder Dimethylsulfoxyd.

Das fertige Reaktionsgemisch besteht im allgemeinen ausschließlich aus zweiwertigem Haptenkonjugat und überschüssigem aktivierten Haptencarboxylat. Die Gewinung des zweiwertigen Haptens erfolgt im allgemeinen durch selektive Ausfällung oder Kristallisation. In schwierigeren Fällen kann es zweckmäßig sein, chromatographische Methoden wie Kieselgelchromatographie anzuwenden.

Es ist anzunehmen, daß solche zweiwertigen Haptene zur Affinitätsreinigung polyklonaler Antikörpergemische dienen könen. Typischerweise enthält ein polyklonales Antikörpergemisch nur einen kleinen Anteil Moleküle, die sich spezifisch mit dem interessierenden Hapten binden. Um diese Fraktion zu isolieren, kann man einen immobilisiertes Hapten enthaltenden Affinitätsträger herstellen. Die Herstellung solcher Träger ist häufig kompliziert, und ihre Leistung hängt von Variablen ab, die nicht leicht zu optimieren sind, wie die Haptenbeladung und die Länge der das Hapten mit der festen Matrix verbindenden "Leine". Eine Übersicht der Methode und der dabei auftretenden Probleme in der Affinitätsreinigung von Antikörpern an einer festen Phase wurden in Immunoadsorbents in Protein Purification, Herausgeber E. Ruoslahti, University Press, Baltimore; Seite 18 (1976) gegeben. Stattdessen kann man kleine lösliche Komplexe zwischen zweiwertigen Haptenen und spezifischen Antikörpern sich bilden lassen, worauf sich die Trennung von fremden Antikörpern nach dem Fachmann wohlbekannten Molekularsiebmethoden erreichen läßt. Nach Säulentrennung werden die Komplexe gemäß dem Stand der Technik wohlbekannten Methoden wie Behandlung mit chaotropen Salzen oder An säuern gespalten. Den freien gereinigten Antiköper erhält man dann durch eine zweite Gelfiltration.

Man kann sich vorstellen, daß eine solche Arbeitsweise wie folgt abläuft:
10 mg polykonales Immunoglobulin G bekannten Titers in phosphatgepufferter Kochsalzösung (PBS), pH 7,4, werden auf eine Konzentration von 1-10 mg/ml eingestellt und mit einem geringen Überschuß zweiwertigem Hapten behandelt. Nach 30-60 Minuten Inkubation bei Raumtemperatur gibt man das Gemisch auf eine mit PBS equilibrierte Säule mit 200 ml Sephadex G-200 Gel. Diese Säule wird mit PBS eluiert, wobei man 1-5 ml-Fraktionen sammelt. Die Komplexe (MG gleich oder größer 300 000) werden vom Gel ausgeschlossen und erscheinen, wenn das "Void-Volume" (V$_o$) der Säule an Flüssigkeit durchgelaufen ist, während die unspezifischen Antikörper später als Peak auftreten. Die Peakfraktionen werden mittels der Extinktion bei 280 nm lokalisiert und vereinigt. Die Komplexe werden durch 5-10 minütige Behandlung mit demselben Volumen 3-molarem Kaliumthiocyanat oder 1-molarer Propionsäure gespalten und dann zur Entfernung von Salzen und freiem zweiwertigem Hapten gelfiltriert.

Die erfindungsgemäßen zweiwertigen Haptenderivate lasen sich auch für turbidimetrische Arzneimittelgehaltsbestimmungen verwenden. In neuerer Zeit ist nämlich eine klinische Testmethode oder Diagnose in den Vordergrund getreten, bei welcher der pathologische Zustand oder die pathologische Prognose eines Patienten beurteilt oder die Arzneimitteldosis, die an den Patenten verabreicht werden soll, in der Weise bestimmt wird, daß der Gehalt des Urins oder des Blutes an einer physiologisch aktiven Substanz mit niederem Molekulargewicht gemessen wird, die in seinem Körper als Hapten und/oder seine Metaboliten oder als ein an den Patienten verabreichtes Arzneimittel und/oder seine Metaboliten vorliegt. Zu messende Haptene sind beispielsweise Schilddrüsen-Hormone wie $T_3$ und $T_4$. Zu messende Arzneimittel sind vor allem folgende: Arzneimittel, deren Dosis möglichst genau ermittelt werden sollte, und deren Wirkungen eine Beziehung zu ihrer Konzentration in dem Blut oder Urin aufweisen, beispielsweise Digitaliszubereitungen, Antibiotika wie Tetracyclin, psychotrope Mittel wie Amphetamin, Narkotika wie Morphin, Blutkoagulationsmittel sowie Antikoagulationsmittel, insbesondere aber Theophyllin und Phenobarbital.

Unter dem Begriff Hapten soll in diesem Zusammenhang eine physiologisch aktive Substanz mit niederem Molekulargewicht verstanden werden, die in dem menschlichen Körper vorliegt, sowie ihre Metabolite, oder ein Arzneimittel, das an den menschlichen Körper verabreicht wird sowie seine Metabolite, wobei eine derartige Substanz allein nicht in der Lage ist, einen Antikörper zu erzeugen, aber dann, wenn sie an eine Trägersubstanz gebunden ist, die selbst ein Antigen ist, wie ein Protein, Polysaccharid oder Glycoprotein, einen Antikörper zu erzeugen vermag.

Gewöhnlich treten die Haptene in Spurenmengen auf, wobei sie als komplexgebundene oder konjugierte Formen in Blut oder Urin mit komplizierter Zusammensetzung vorliegen. Daher sind die Verfahren zu ihrer Ermittlung und Messung aufwendig und zeitraubend.

Um diese Haptene zu messen, kann man sich verschiedener Methoden bedienen, und zwar physikalisch-chemischer oder immunchemischer Verfahren oder konkurrierender Proteinbindeverfahren.

Immunchemische Verfahren sind mittlerweile bezüglich der Reaktionsspezifität und der Meßempfindlichkeit den physikalisch-chemischen Verfahren überlegen. Derzeit sind zahlreiche immunchemische Methoden zur Messung von Haptenspuren im menschlichen Körper bekannt, beispielsweise Agglutinationshemmungsverfahren, der Radioimmunoassay (nachfolgend als RIA abgekürzt) sowie der Enzymimmunoassay (nachfolgend als EIA bezeichnet). Bei einem bekannten EIA-Verfahren werden mit einem Antikörper zu dem zu messenden Hapten sensibilisierte Teilchen und ein Kopplungsprodukt des Haptens mit einer Trägersubstanz verwendet (DE-OS 21 55 658).

Bei der Durchführung eines Agglutinationshemmungsverfahrens kann ein Kopplungsprodukt aus dem zu messenden Hapten und einer Trägersubstanz wie einem Protein, einem Polysaccharid oder einem Glycoprotein mit hohem Molekulargewicht gebunden an feste Teilchen, zum Beispiel Blutzellen oder Latexteilchen, als antigene Komponente verwendet werden. Ein Antikörper zu dem zu messenden Hapten wird aus einem Antiserum erhalten, das aus Säugetieren gewonnen wird, beispielsweise Meerschweinchen, Kaninchen oder Schafen, die ebenfalls mit einem Kopplungsprodukt aus dem Hapten und einer Trägersubstanz immunisiert worden sind.

Wird der Antikörper mit den Teilchen, an welche das Haptenträgerkonjugat gekoppelt ist, vermischt, dann erfolgt eine Agglutinationsreaktion zwischen diesen zwei Komponenten. Die Agglutinierungsreaktion wird inhibiert, wenn die Probe das zu messende Hapten enthält. Dies ist ein einfaches Verfahren, das in der Lage ist, das in Form eines Komplexes oder Konjugates in Blut oder Urin vorliegende Hapten zu messen, ohne daß dabei komolizierte Verfahrensmaßnahmen wie eine Hydrolyse oder Chromatographie erforderlich sind. Die Empfindlichkeit beträgt ungefähr 100 ml, und zwar auch dann, wenn Blutzellen als Teilchen verwendet werden. Da aber die meisten Haptene, deren Messung im menschlichen Körper von Interesse ist, in einer Menge zwischen 500 pg/ml und 50 ng/ml vorliegen, müssen sie konzentriert werden.

Eine weitere Aufgabe der Erfindung ist daher die Schaffung eines neuen immunchemischen Haptenmeßverfahrens mittels Agglutinationshemmung, das eine höhere Meßempfindlichkeit als die bekannten Methoden aufweist.

Diese Aufgabe wird gelöst mit einem Verfahren zur immunchemischen Messung eines Haptens mittels Agglutinationshemmung, dadurch gekennzeichnet, daß mit einem gegen das zu messende Hapten gerichteten Antikörper sensibilisierte Teilchen, eine niedermolekulare, die Haptengruppe zweifach enthaltende Verbindung und die das zu messende Hapten enthaltende Flüssigkeit zusammengebracht und aus der durch das Hapten verursachten Turbiditätsverringerung die Menge des Haptens bestimmt wird.

Es ist Stand der Technik, daß bei einer Agglutinationshemmungsmethode (Patentschrift DE 27 43 445) mit einem Antikörper zu dem zu bestimmenden Hapten sensibilisierte Teilchen verwendet werden sowie ein mit einem Träger gekoppeltes Hapten.

Zur Durchführung des erfindungsgemäßen Bestimmungsverfahrens wird gewöhnlich ein bestimmtes Volumen der Körperflüssigkeit oder des Exkretes, welche oder welches das zu messende Hapten enthält, direkt oder auf verschiedene Konzentrationen verdünnt, in die Reaktion mit einem gegebenen Volumen einer mit dem Antikörper sensibilisierten Teilchensuspension eingesetzt.

Dann wird ein gegebenes Volumen des bifunktionellen Haptens ("Di-Haptens") dieser Reaktionsmischung zugesetzt. Nach einer bestimmten Reaktionszeitspanne wird eine nephelometrische oder turbidimetrische Messung durchgeführt. Die Auswertung erfolgt anhand einer Referenzkurve.

Die Antikörper zu dem Hapten werden in bekannter Weise wie folgt erhalten: Ein Hapten wird an eine Trägersubstanz gekoppelt. Ein Antiserum wird aus einem Tier erhalten, das nach einer Routinemethode mit dem mit dem Träger gekoppelten Hapten als Antigen immunisiert worden ist. Die Antikörper zu dem Hapten werden in der Weise gewonnen, daß durch Absorption die anderen Antikörper, das heißt die gegen den Träger oder gegen die Bindungsstelle zwischen dem Träger und dem Hapten gerichteten Antikörper entfernt werden. Als Trägersubstanzen kann man viele Materialien verwenden. Gute Ergebnisse werden erhalten, wenn die Trägersubstanzen eine hohe Antigenwirkung aufweisen beispielsweise Keyhole Limpet Hemocyanin.

Als zu sensibilisierende Teilchen sind feinteilige Träger, wie sie normalerweise zur Durchführung in immuchemischen Agglutinationsreaktionen oder Agglutinationshemmungsreaktionen eingesetzt werden wie hochmolekularer Latex zu verwenden.

Zur Sensibilisierung von Latexteilchen kann der Antikörper zu dem nach den vorstehend beschriebenen Methoden erhaltenen Hapten nach dem Verfahren der europäischen Patentanmeldung 0080614 (USP 4,448,908 und Certificate of Correction) kovalent an die Teilchen gebunden werden.

Das zur Erzeugung eines Antikörpers zu dem Hapten eingesetzte mit einem Träger gekoppelte Hapten sowie das als sogenannter "Developer" im Meßverfahren verwendete eingesetzte Dihapten können an der Kopplungsstelle zwischen der Trägersubstanz und dem Hapten voneinander verschieden sein.

Mit dem erfindungsgemäßen Verfahren meßbare Haptene sind beispielsweise die Arzneimittel Theophyllin, Phenobarbital, Diphenylhydantoin, Digoxin, Primidon, Valproinsäure, Carbamazepin, Gentamycin, Tobramycin oder Kanamycin.

Weitere solche Haptene sind zum Beispiel Schilddrüsenhormone wie $T_3$ und $T_4$.

Im Vergleich zu der herkömmlichen Methode bietet die Erfindung eine außergewöhlich hohe Meßempfindlichkeit.

Im erfindungsgemäßen Verfahren sind nur kleine Mengen an dem Antikörper zu dem Hapten und dem Dihapten erfoderlich. Beispielsweise kann der Verbrauch an dem Dihapten Di-Theophyllin auf ungefähr 1/100 bis 1/1000 der Menge herabgesetzt werden, die in der herkömmlichen Methode erforderlich ist.

Nach der in DE 27 43 445 C2 beschriebenen Methode zur Messung von Haptenen durch Agglutinationshemmung wird ein Hapten, gekoppelt an einen Träger, verwendet. Wie dort beschrieben, läßt sich die Meßempfindlichkeit noch weiter erhöhen, wenn das Haptenträgerkonjugat an feine Teilchen gebunden ist.

Wir haben überraschenderweise gefunden, daß sich ein dimeres Haptenderivat besser für eine nephelometrische Messung eignet als ein Hapten-Träger-Konjugat.

Die Vorteile des dimeren Haptenderivates liegen darin, daß die Messung empfindlicher ist und in kürzerer Zeit durchgeführt werden kann. Ein weiterer großere Vorteil liegt darin, daß die aufwendige Synthese eines Hapten-Träger-Konjugates wie zum Beispiel eines Hapten-Apo ferrins oder Hapten-Albumin-Konjugates entfällt. Wie dem Fachmann geläufig ist, sind die genannten Protein-Hapten-Konjugate aufgrund von Nebenreaktionen nur mit mäßiger Ausbeute herzustellen und die Qualität schwankt. Diese Derivate sind sehr heterogen in Bezug auf Molekulargewicht (Protein-Protein-Vernetzung) und Menge der Beladung mit Hapten. Die Herstellung der dimeren Haptenderivate ist einfach und kostengünstig. Da keine unkontrollierten Nebenreaktionen auftreten können, ist die Qualitätskonstanz gewährleistet und sicher mit Hilfe analytischer Daten (massenspektroskopische Analyse, C-H-N-Analyse, Infrarot- oder Kernresonanzspektrum) überprüfbar.

Die nachfolgenden Beispiele erläutern die Erfindung.

Beispiel 1

Theophyllin-8-buttersäure

Man erhitzt 25 g Diaminodimethyluracilhydrat und 50 g Glutarsäureanhydrid 3 Stunden lang in 250 ml Dimethylanilin unter Rückfluß. Dann kühlt man ab und gibt das Gemisch zu 1,5 Litern Wasser und 50 ml konzentrierter Natronlauge. Die so erhaltene Lösung wird zur Entfernung des Dimethylanilins mit 300 ml Ether extrahiert. Die wässrige Schicht verdünnt man mit 1,5 Litern Wasser und leitet sie dann durch eine Säule mit 1,5 Litern Dowex-2-Anionenaustauscherharz in der Formiatform. Die Säule wird mit 6 Litern Wasser und dann 4 Litern 0,2 molarer Ameisensäure in einem 1:1-Gemisch aus Wasser und Aceton eluiert. Die produkthaltigen Eluate werden vereinigt und bei vermindertem Druck zu einem Kristallbrei eingedampft.

Man sammelt die Kristalle, wäscht mit kaltem Wasser und kristallisiert aus heißem Isopropanol um, wobei man 20 g Theophyllin-8-buttersäure erhält.

Bis-(theophyllin-8-butyryl)-1,6-hexandiamid (Vergleichsbindung)

Man löst 1 g Theophyllin-8-buttersäure in 10 ml Dimethylformamid, gibt 0,89 ml Tributylamin dazu und kühlt die Lösung auf 5°C. Dann setzt man 0,48 ml Chlorameisensäureisobutylester zu, rührt 10 Minuten lang und versetzt mit 0,2 g in 10 ml Dimethylformamid gelöstem und auf 5°C gekühltem Hexandiamin. Das Reaktionsgemisch wird 30 Minuten lang bei 0-5°C gerührt. Dabei bildet sich ein schwerer gelatinöser Niederschlag, der abfiltriert und mit Dimethylformamid gewaschen wird. Nach Trocknen des Produkts im Vakuum erhält man 0,75 g Bis-(theophyllin-8-butyryl)-1,6-hexandiamid.

Bis-(theophyllin-8-butyryl)-4,9-dioxal-1,12-dodecandiamid

Die Synthese wird unter Ersatz des 1,6-Hexandiamins durch 0,4 ml 4,9-Dioxa-1,12-dodecandiamin durchgeführt. Das Reaktionsgemisch, eine Lösung, wird unter Rühren in 500 ml Ethyläther gegossen. Nach Kühlung über Nacht (ungefähr 16 Stunden) filtriert man die Suspension. Der rohe Feststoff wird aus Methanol umkristallisiert, was 0,45 g Bis-(theophyllin-8-butyryl)-4,9-dioxa-1,12-dodecandiamid ergibt.

Löslichkeitsvergleich der Bis-(theophyllin-8-butyryl)-diamide

Die Bis-(theophyllin-8-butyryl)-diamide des 1,6-hexandiamins bzw. 4,9-dioxa-1,12-dodecandiamins werden jeweils in Dimethylsulfoxyd gelöst. Das 4,9-Dioxa-1,12-dodecandiamid löst sich leicht bei Raumtemperatur zu einer Lösung von 10 mg/0,5 ml. Beim Verdünnen dieser Lösung mit 2 ml 50-millimolarem wässrigen Natriumphosphatpuffer (pH 7,5) erhält man eine klare stabile Lösung. Dagegen erfordert das 1,6-Hexandiamid Erhitzen, um eine Lösung von 10 mg in 2 ml Dimethylsulfoxyd zu erhalten, und beim Verdünnen dieser Lösung mit einem zweifachen Volumen Phosphatpuffer von pH 7,5 beobachtet man eine sofortige umfangreiche Ausfällung des zweiwertigen Haptens.

Beispiel 2

Theophyllin-8-buttersäurelactam

Man löst 24 g Theophyllin-8-buttersäure (Beispiel 1) unter Erhitzen in 500 ml Essigsäureanhydrid. Nach 30 Minuten Erhitzen unter Rückfluß filtriert man das Gemisch durch ein vorgeheiztes Filter. Das Produkt kristallisiert beim Abkühlen, wird gesammelt, mit Ether und Hexan gewaschen und bei 50°C im Vakuum getrocknet, was 20 g Theophyllin-8-buttersäurelactam ergibt.

Bis-(theophyllin-8-butyryl)-N,N-bis-3-aminopropylpiperazin-diamid

Man suspendiert 0,5 g Theophyllin-8-buttersäurelactam in 5 ml Dimethylformamid. Dazu gibt man unter Rühren 0,2 ml Bis-(3-aminopropyl)-piperazin und 0,48 ml Tributylamin. Dies ergibt zunächst eine Lösung, aber nach 15 Minuten bildet sich ein schwerer gelatinöser Niederschlag. Das Reaktionsgemisch wird mit 5 ml Dimethylformamid verdünnt und über Nacht gerührt. Nach Abfiltrieren des Niederschlags, Waschen mit Dimethylformamid und Trocknen erhält man 0,6 g Bis-(theophyllin-8-butyryl)-N,N-bis-3-aminopropylpiperazin.

Beispiel 3

Phenobarbital-N-propionsäure

Man gibt 93 g Phenobarbital und 74 g 3-Brompropionsäure in 200 ml Wasser. Unter Durchmischen setzt man dann 48 g Natriumhydroxyd zu. Man erhitzt unter gelegentlichem Umschwenken 1,5 Stunden lang auf einem Dampfbad. Dann gibt man weitere 16 g Natriumhydroxyd zu und erhitzt weitere 1,5 Stunden. Die Lösung wird mit Wasser auf 1,6 Liter verdünnt und mit konzentrierter Salzsäure auf pH 6,5 gestellt. Dabei bildet sich ein Niederschlag aus nicht umgesetztem Phenobarbital. Zur weitgehenden Entfernung des Phenobarbitals wird das Gemisch mit dreimal je 400 ml Essigester extrahiert. Die verbleibende wässrige Lösung stellt man mit konzentrierter Salzsäure auf pH 4,5, was eine milchige Suspension liefert. Diese wird mit zweimal 400 ml Essigester extrahiert. Den vereinigten produkthaltigen Extrakt wäscht man mit Wasser und gesättigter Kochsalzlösung. Der Extrakt wird über Magnesiumsulfat getrocknet, filtriert und zu einem Öl eingedampft. Das Öl wird durch Auflösen in Aceton und Zusatz von Hexan bis zur Trübung kristallisiert. Das Produkt wird gesammelt und getrocknet, was 20 g Phenobarbital-N-propionsäure ergibt.

Bis-(phenobarbital-N-propionyl)-4,9-dioxa-1,12-dodecandiamid

Man löst 1,27 g Phenobarbital-N-propionsäure in 5 ml Dimethylformamid. Unter Rühren versetzt man tropfenweise bei Raumtemperatur mit 0,61 g in 5 ml Dimethylformamid gelöstem Carbonyldiimidazol. Nach 30 Minuten tropft man 0,40 ml 4,9-Dioxa-1,12-dodecandiamin in 5 ml Dimethylformamid zu. Dann rührt man 2 Stunden lang bei Raumtemperatur. Das Lösungsmittel wird im Hochvakuum abgedampft und der Rückstand mit Hexan angerieben, was einen Feststoff liefert. Das so erhaltene Rohprodukt löst man in 10 ml Chloroform/Methanol (9:1) und gibt es auf eine Kieselgelsäule. Die Säule wird mit Chloroform und danach mit Chloroform/Methanolgemischen im Bereich von 9:1 bis 1:1 eluiert. Das mit 9:1-Gemisch eluierte Produkt ist gemäß Dünnschichtchromatographie (DC) einheitlich. Die Fraktionen werden vereinigt und zu einem Öl eingedampft. Trocknen im Vakuum liefert 0,65 g Bis-(phenobarbital-N-propionyl)-4,9-dioxa-1,12-dodecandiamid als amorphen Feststoff.

Beispiel 4

Trifluoracetyl-L-thyroxin

Man gibt 10 g L-Thyroxin-natriumsalz unter Rühren zu einem gekühlten Gemisch aus 150 ml Esssigester und 40 ml Trifluoressigsäure. Die erhaltene Lösung versetzt man tropfenweise mit 10 ml Trifluoressisäureanhydrid. Nach beendeter Zugabe rührt man 2 Stunden lang bei 0-2°C, gießt dann in 200 ml eiskalte Kochsalzlösung, verdünnt das Gemisch mit 100 ml Essigester und trennt die Phasen.

Die Essigesterphase wird mit 1-molarem Natriumbisulfat und dann mit gesättigter Kochsalzlösung gewaschen. Dann trocknet man die Lösung über Magnesiumsulfat, filtriert und engt auf ein kleines Volumen ein. Verdünnen mit Ether und danach mit niedrig siedendem Petrolether ergibt einen flockigen Niederschlag, der gesammelt und getrocknet wird, wobei man 8,2 g Produkt erhält. In einer DC zeigt sich ein geringfügiger zweiter Fleck von Thyroxin. Reinigung durch Kieselgelchromatographie in Chloroform/Methanol (9:1) ergibt 4,3 g Trifluoracetyl-L-thyroxin.

Bis-(trifluoracetyl-L-thyroxyl)-4,9-dioxa-1,12-dodecandiamid

Man löst 1,23 g Trifluoracetyl-L-thyroxin in 8 ml Tetrahydrofuran und kühlt die Lösung im Eis/Acetonbad auf -2°C. Man setzt 0,16 ml N-Methylmorpholin und danach 0,18 ml Chlorameisensäureisobutylester zu. Nach 7 Minuten Rühren wird eine Lösung von 0,15 ml 4,9-Dioxa-1,12-dodecandiamin in 2 ml Tetrahydrofuran im Verlauf von 5 Minuten zugetropft. Man rührt 30 Minuten lang bei 0°C und erwärmt dann auf Raumtemperatur. Zur Entfernung von Salzen wird das Reaktionsgemisch filtriert, und das Filtrat wird zu einem Öl eingedampft.

Dieses löst man in Essigester wieder auf und wäscht die Lösung mit wässrigem 1-molaren Natriumbisulfat und dann mit 0,5-molarem Natriumchlorid. Die Essigesterlösung wird über Magnesiumsulfat getrocknet, filtriert und zu einem Öl eingeengt. Das Öl kristallisiert man aus Essigester/Hexan, was 0,6 g Bis-(trifluoracetyl-L-thyroxyl)-4,9-dioxa-1,12-dodecandiamid in zwei Kristallisaten ergibt.

Bis-(L-thyroxyl)-4,9-dioxa-1,12-dodecandiamid-dihydrochlorid

Man löst 0,34 g Bis-(trifluoracetyl-L-thyroxyl)-4,9-dioxadodecan-1,12-diamid in 3 ml Methanol. Unter Rühren versetzt man mit 1-molarer Natronlauge und läßt die Umsetzung 40 Stunden lang bei Raumtemperatur ablaufen. Dann verdünnt man mit 20 ml kalter 1-molarer Salzsäure, was eine feine Suspension des Produkts ergibt. Man zentrifugiert die Suspension und löst den Niederschlag in Ethanol. Die Ethanollösung wird zu einem festen Rohproduktrückstand eingedampft. Das Produkt wird aus Methanol/Ether umkristalliert, wobei man 0,18 g Bis-(L-thyroxyl)-4,9-dioxa-1,12-dodecandiamid-dihydrochlorid erhält.

Beispiel 5

Bis-(N-succinyl-L-thyroxyl)-4,9-dioxa-1,12-dodecandiamid

Man tropft eine lösung von 2 g Bernsteinsäureanhydrid in 20 ml Pyridin in eine gekühlte Lösung von 2 g 4,9-Dioxa-1,12-dodecandiamin in 20 ml Pyridin. Man läßt das Reaktionsgemisch 18 Stunden lang bei 4°C stehen und dampft es dann zu einem festen Rückstand ein. Dieser wird in 30 ml Wasser gelöst und durch eine Säule mit 30 ml Dowex 50-Kationenaustauscher in der $H^+$-Form geleitet. Dann wäscht man die Säule mit 60 ml Wasser. Der Durchlauf wird auf etwa das halbe Volumen eingeengt, was Bissuccinyl-4,9-dioxa-1,12-dodecandiamid als farblose Kristalle liefert, die gesammelt und getrocknet werden. Die Ausbeute beträgt 2,18 g.

Das Zwischenprodukt löst man in 30 ml Dimethylformamid, zusammen mit 1,5 g N-Hydroxysuccinimid. Die Lösung wird abgekühlt und auf einmal mit 2,45 g Dicyclohexylcarbodiimid in 15 ml Dimethylformamid versetzt. Nach 18 Stunden bei 4°C filtriert man das Reaktionsgemisch und dampft das Filtrat zu einem Öl ein. Dieses wird aus Isopropanol und Ether kristallisiert, was 1,3 g Bis-succinimido-O,N-succinyl-4,9-dioxa-1,12-dodecandiamin als kristallines Produkt liefert. 0,3 g dieses aktiven Esters werden in 2 ml Dimethylformamid gelöst und tropfenweise zu einer Suspension von 0,89 g L-Thyroxin-Natriumsalz in 4 ml Dimethylformamid, das 0,11 ml N-Methylmorpholin enthält, gegeben. Man rührt das Reaktionsgemisch 72 Stunden lang und dampft die erhaltene Lösung zu einem Öl ein. Das Öl wird mit einem Gemisch aus Essigester und 1-molarem Natriumbisulfat gewaschen, was einen Feststoff liefert, der durch Filtrieren gesammelt, mit Wasser und mit Methanol gewaschen und getrocknet wird, wobei man 0,22 g Bis-(N-succinyl-L-thyroxyl)-4,9-dioxa-1,12-dodecandiamid erhält.

Beispiel 6

Bestimmung von Theophyllin

Als Dihapten wurde N,N'-Bis-(theophyllin-8-butyrylamino-3-propyl)-piperazin eingesetzt.

Das Dihapten wurde in Dimethylsulfoxid in einer Konzentration von 1 mg/ml gelöst und anschließend 1:51200 in destilliertem Wasser mit Zusatz von 4 g/100 ml Human-Serum-Albumin verdünnt. Das verdünnte Dihapten fungiert im Test als "Developer".
Es wurde ein Latexreagenz durch Bindung von Antiserum gegen Theophyllin an Polystyrollatex hergestellt. Die Herstellung des Anti-Theophyllin-Latex-Reagenzes erfolgte wie in EP 0080614 beschrieben.

Zur Messung wurden in einer Küvette 10 µl einer Theophyllin enthaltenden Probe, 1:100 verdünnt in einem 0,1 m Glycin-NaCl-Puffer pH 8,0, mit 10 µl der oben beschriebenen Developer-Verdünnung sowie mit 20 µl einer 4,6 ml/100 ml Polyethylenglycol 6000 enthaltenden phosphatgepufferten Kochsalzlösung und mit 10 µl des oben beschriebenen Anti-Theophyllin-Latex-Reagenzes gemischt. Die Mischung wurde 12 min lang bei Raumtemperatur inkubiert. Anschließend wurden weitere 200 µl einer 4,6 ml/100 ml Polyethylenglycol 6000 enthaltenden phosphatgepufferten Kochsalzlösung hinzugegeben, gemischt und die Lichtstreuung an einem Nephelometer gemessen.

Mit unterschiedlichen Mengen des hinzugegebenen Theophyllins wurde eine Inhibitionskurve erstellt. Der Meßbereich erstreckt sich von etwa 1 bis 40 µg/ml Theophyllin.

Zum Vergleich wurde eine Messung mit einem Apoferritin-Developer nach Stand der Technik durchgeführt. Das Apoferritin-Theophyllin-Derivat wurde von der Firma Kallestad bezogen (Chargennummer: R 1). Es wurde 1:1280 in wäßriger Human-Serum-Albumin-Lösung verdünnt und in dieser Verdünnung im Test als Developer eingesetzt. Der Ansatz zur Messung wurde wie zuvor beschrieben durchgeführt, mit der Ausnahme, daß das Apoferritin-Theophyllin-Derivat an Stelle des Dihapten-Developers eingesetzt wurde. Die mit unterschiedlichen Mengen an hinzugegebenem Theophyllin erhaltene Inhibitionskurve zeigte eine geringere Empfindlichkeit, das heißt die untere Nachweisgrenze lag höher als nach der Erfindung.

Beispiel 7

Messung von Phenobarbital

Als Dihapten wurde 1,12-Bis-(phenobarbital-propionylamino)-4,9-dioxa-dodecan eingesetzt.

Das Dihapten wurde in Dimethylsulfoxid in einer Konzentration von 1 mg/ml gelöst und anschließend 1:40 000 in wäßriger 4 g/100 ml Human-Serum-Albumin-Lösung verdünnt. Das verdünnte Dihapten fungiert im Test als "Developer".

Es wurde ein Latexreagenz durch Bindung von Antiserum gegen Phenobarbital an Polystyrollatex hergestellt. Die Herstellung des Anti-Phenobarbital-Latex Reagenzes erfolgte wie in EP 0080614 beschrieben.

Zur Messung wurden in einer Küvette 20 µl einer Phenobarbital enthaltenden Probe, 1:100 verdünnt in einem 0,1-molaren Glycin-NaCl-Puffer, pH 8,0, mit 20 µl der oben beschriebenen Developer-Verdünnung sowie mit 40 µl einer 4,6 ml/100 ml Polyethylenglycol 6000 enthaltenden phosphatgepufferten Kochsalzlösung und mit 20 µl des oben beschriebenen Anti-Phenobarbital-Latex Reagenzes gemischt. Die Mischung wurde 25 min lang bei Raumtemperatur inkubiert. Anschließend wurden weitere 200 µl einer 4,6 ml/100 ml Polyethylenglycol 6000 enthaltenden phosphatgepufferten Kochsalzlösung hinzugegeben, gemischt und die Lichtstreuung an einem Nephelometer gemessen. Mit unterschiedlichen Mengen an hinzugegebenem Phenobarbital wurde eine Inhibitionskurve erstellt. Der Meßbereich erstreckt sich von etwa 2,5 bis 80 µg/ml Phenobarbital.

Zum Vergleich wurde eine Messung mit einem Apoferritin-Developer nach Stand der Technik durchgeführt. Das Apoferritin-Phenobarbital-Derivat wurde von der Firma Kallestad bezogen (Chargennummer 026-S1). Es wurde 1:750 in wäßriger Human-Serum-Albumin-Lösung verdünnt und in dieser Verdünnung im Test als Developer eingesetzt. Der Ansatz zur Messung wurde wie zuvor beschrieben durchgeführt. An Stelle des Dihapten-Developers wurde das Apoferritin-Phenobarbital-Derivat eingesetzt. Mit unterschiedlichen Mengen an hinzugegebenem Phenobarbital ergab sich eine sehr flache Inhibitionskurve, die aus diesem Grunde nicht für eine gut reproduzierbare Bestimmung verwendbar ist.

## Patentansprüche

1. Verbindung, die aus zwei über ein bifunktionelles Brückenglied verbundenen Haptenkomponenten besteht, wodurch diese Verbindung der Formel I

   A-X-A     I

   entspricht, worin A eine Haptenkomponente und X ein bifunktionelles Brückenglied der Formel II

   $(B)_m$-Y-$(CH_2)_n$-Z-$(CH_2)_n$-Y-$(B)_m$     II

   bedeuten, worin die Indices m unabhängig voneinander 0 oder 1 sind, B $(CH_2)_{n'}$ mit n' als ganze Zahl von 1 bis 4 oder $CO(CH_2)_{n''}$ mit n'' als ganze Zahl von 2 bis 4 bedeutet,

   die Reste Y unabhängig voneinander -CONH-, -NHCO-, -OOC-, -COO-, -O-, -S- oder -NR- sind, wobei R für Wasserstoff oder einen aliphatischen Rest mit bis zu 6 C-Atomen steht,

   n eine ganze Zahl von 1 bis 10 und

   Z -O-$(CH_2)_4$-O-, Piperaz -1,4-diyl oder -NH-$(CH_2)_4$-NH-ist.

2. Verbindung nach Anspruch 1, dadurch gekennzeichnet, daß die Haptenkomponente Theophyllin ist.

3. Verbindung nach Anspruch 1, dadurch gekennzeichnet, daß die Haptenkomponente L-Thyroxin ist.

4. Verbindung nach Anspruch 1, dadurch gekennzeichnet, daß die Haptenkomponente Phenobarbital ist.

5. Verwendung einer Verbindung nach Anspruch 1 zur Affinitätsreinigung polyklonaler Antikörpergemische.

6. Verwendung einer Verbindung nach Anspruch 1 zur Haptenbestimmung mittels einer Trübungsmessung.

7. Immunchemisches Haptenbestimmungsverfahren mittels Agglutinationshemmung, dadurch gekennzeichnet, daß man ein mit einem Antikörper zu dem zu bestimmenden Hapten sensibilisiertes Teilchen, eine Verbindung nach Anspruch 1 sowie eine Flüssigkeit, in welches das Hapten bestimmt werden soll, zusammenbringt, die durch das Hapten verursachte und von der Konzentration des Haptens in der Flüssigkeit abhängige Turbiditätsverringerung mit und daraus die Konzentration des Haptens in der Flüssigkeit bestimmt.

8. Verfahren nach Anspruch 7, dadurch gekennzeichnet, daß die den Antikörper tragenden Teilchen aus einem hochmolekularen Latex bestehen.

9. Verfahren nach Anspruch 7, dadurch gekennzeichnet, daß der Antikörper kovalent an einen Latex gebunden ist.

10. Verfahren nach Anspruch 7, dadurch gekennzeichnet, daß der Antikörper durch Immunisieren eines Tieres mit einem Kopplungsprodukt von Keyhole Limpet Hemocyanin und dem Hapten gewonnen wurde.

11. Verfahren nach Anpsruch 7, dadurch gekennzeichnet, daß trübungsphotometrisch gemessen wird.

## Claims

1. A compound which is composed of two hapten components connected by means of a bifunctional bridging member, as a result of which this compound corresponds to the formula I

   A-X-A     I

   in which A is a hapten component and X is a bifunctional bridging member of the formula II

   $(B)_m$-Y-$(CH_2)_n$-Z-$(CH_2)_n$-Y-$(B)_m$     II

   in which, independently of one another, the indices m are 0 or 1 and B is $(CH_2)_{n'}$ with n' as an integer from 1 to 4 or $CO(CH_2)_{n''}$ with n'' as an integer from 2 to 4,

   the radicals Y independently of one another are -CONH-, -NHCO-, -OOC-, -COO-, -O-, -S- or -NR-, where R is hydrogen or an aliphatic radical having not more than 6 carbon atoms,

   n is an integer from 1 to 10 and

   Z is -O-$(CH_2)_4$-O-, piperazine-1,4-diyl or -NH-$(CH_2)_4$-

2. A compound as claimed in claim 1, wherein the hapten component is theophylline.

3. A compound as claimed in claim 1, wherein the hapten component is L-thyroxine.

4. A compound as claimed in claim 1, wherein the hapten component is phenobarbital.

5. The use of a compound as claimed in claim 1 for affinity purification of polyclonal antibody mixtures.

6. The use of a compound as claimed in claim 1 for hapten determination by means of a turbidity measurement.

7. A method for immunochemical determination of haptens by means of inhibition of agglutination, wherein a particle sensitized with an antibody to the hapten to be determined, a compound as claimed in claim 1 and a liquid in which the hapten is to be determined are combined, the reduction of turbidity produced by the hapten and dependent on the concentration of the hapten in the liquid is determined and, from this, the concentration of the hapten in the liquid is determined.

8. A method as claimed in claim 7, wherein the particles carrying the antibodies are composed of a high molecular weight latex.

9. A method as claimed in claim 7, wherein the antibody is covalently bonded to a latex.

10. A method as claimed in claim 7, wherein the antibody has been obtained by immunization of an animal with a coupling product of keyhole limpet hemocyanin and the hapten.

11. The method as claimed in claim 7, wherein the measurement is carried out turbidimetrically.

**Revendications**

1. Composé constitué de deux composants haptène liés par un chaînon pontant bifonctionnel, ce composé correspondant à la formule I

A-X-A     I

dans laquelle A représente un composant haptène et X représente un chaînon pontant bifonctionnel de formule II

$(B)_m$-Y-$(CH_2)_n$-Z-$(CH_2)_n$-Y-$(B)_m$     II

dans laquelle les indices m, indépendamment l'un de l'autre, sont 0 ou 1, B représente $(CH_2)_{n'}$ n' étant un nombre entier allant de 1 à

4, ou $CO(CH_2)_{n''}$ n'' étant un nombre entier allant de 2 à 4,
les radicaux Y représentent, indépendamment l'un de l'autre, -CONH-, -NHCO-, -OOC-, -COO, -O-, -S- ou -NR-, R représentant un atome d'hydrogène ou un radical aliphatique ayant jusqu'à 6 atomes de carbone,
n est un nombre entier allant de 1 à 10, et Z représente un groupe -O-$(CH_2)_4$-O-, pipéraz-1,4-diyle ou -NH-$(CH_2)_4$-NH-.

2. Composé selon la revendication 1, caractérise en ce que le composant haptène est la théophylline.

3. Composé selon la revendication 1, caractérisé en ce que le composant haptène est la L-thyroxine.

4. Composé selon la revendication 1, caractérisé en ce que le composant haptène est le phénobarbital.

5. Utilisation d'un composé selon la revendication 1, pour la purification par affinité de mélanges d'anticorps polyclonaux.

6. Utilisation d'un composé selon la revendication 1, pour le dosage d'haptène au moyen d'une mesure de la turbidité.

7. Procédé immunochimique de dosage d'haptène par inhibition d'agglutination, caractérisé en ce que l'on met en contact une particule sensibilisée avec un anticorps dirigé contre l'haptène à déterminer, un composé selon la revendication 1 ainsi qu'un liquide dans lequel on désire doser l'haptène, et on détermine la diminution de turbidité provoquée par l'haptène et dépendant de la concentration de l'haptène dans le liquide, et à partir de celle-là, la concentration de l'haptène dans le liquide.

8. Procédé selon la revendication 7, caractérisé en ce que les particules portant l'anticorps sont constituées d'un latex à masse moléculaire élevée.

9. Procédé selon la revendication 7, caractérisé en ce que l'anticorps est fixé par liaison covalente à un latex.

10. Procédé selon la revendication 7, caractérisé en ce que l'anticorps a été obtenu par immunisation d'un animal avec un produit de couplage d'hémocyanine de patelle et de l'haptène.

11. Procédé selon la revendication 7, caractérisé en ce que l'on effectue la mesure par turbidimétrie.